(19) Europäisches Patentamt
European Patent Office
Office européen des brevets

(11) Veröffentlichungsnummer : **0 460 446 B1**

(12) # EUROPÄISCHE PATENTSCHRIFT

(45) Veröffentlichungstag der Patentschrift :
**07.04.93 Patentblatt 93/14**

(51) Int. Cl.$^5$ : **C07C 275/70,** C07C 273/18

(21) Anmeldenummer : **91108125.5**

(22) Anmeldetag : **21.05.91**

(54) **Ein neues Kupplungsreagenz für die Peptidsynthese.**

(30) Priorität : **23.05.90 DE 4016596**

(43) Veröffentlichungstag der Anmeldung :
**11.12.91 Patentblatt 91/50**

(45) Bekanntmachung des Hinweises auf die Patenterteilung :
**07.04.93 Patentblatt 93/14**

(84) Benannte Vertragsstaaten :
**AT BE CH DE DK ES FR GB GR IT LI LU NL SE**

(56) Entgegenhaltungen :
**Keine Entgegenhaltungen.**

(73) Patentinhaber : **HOECHST AKTIENGESELLSCHAFT**
**Postfach 80 03 20**
**W-6230 Frankfurt am Main 80 (DE)**

(72) Erfinder : **Breipohl, Gerhard, Dr.**
**Geisenheimer Strasse 95**
**W-6000 Frankfurt am Main (DE)**
Erfinder : **König, Wolfgang, Dr.**
**Eppsteiner Strasse 25**
**W-6238 Hofheim am Taunus (DE)**

EP 0 460 446 B1

Anmerkung : Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

## Beschreibung

Die Erfindung betrifft ein Kupplungsreagenz für die Peptidsynthese.

Aus Synthesis, 1984, 572 - 574 und Tetrahedron Letters, Vol. 30, No. 15 (1989), 1927 - 1939 sind Uronium Salze bekannt, wie 2-(1H-Benzotriazol-1-yl)-1,1,3,3-tetramethyluronium-hexafluorphosphat (HBTU), 2-(1H-Benzotriazol-1-yl)-1,1,3,3-tetramethyluronium-hexafluorborat (TBTU), 2-(3,4-Dihydro-4-oxo-1,2,3-benzotriazin-3-yl)-1,1,3,3-tetramethyluronium-tetrafluorborat (TDBTU), 2-(2-Oxo-1[2H]-pyridyl)-1,1,3,3-tetramethyluronium-tetrafluorborat (TPTU), 2-Succinimido-1,1,3,3-tetramethyluronium-tetrafluorborat (TSTU), 2-(5-Norbornen-2,3-dicarboximido)-1,1,3,3-tetramethyluronium-tetrafluorborat (TNTU). Ebenfalls bekannt ist 2-Hydroxyimino-2-cyanoessigsäure-ethylester und seine Verwendung als Additiv zur Racemisierungsunterdrückung bei Peptidkupplungen (Masumi Itoh, Peptides: Chemistry and Biology of Peptides, Proceedings of the fourth American Peptide Symposium, Seiten 365 -367, Hrsg: R. Walter, J. Meienhofer, Ann Arbor Science Publishers 1972).

Da bei der Herstellung von bekannten Uronium-Salzen, wie TBTU, die Verwendung von gesundheitsgefährdendem und toxischem Dichlormethan und Acetonitril unerläßlich ist, liegt der Erfindung die Aufgabe zugrunde neue Kupplungsreagenzien für die Peptidsynthese zu finden, die sich u.a. durch ein einfaches Herstellungsverfahren auszeichnen.

Diese Aufgabe wird erfindungsgemäß gelöst durch die Verbindungen der Formel I

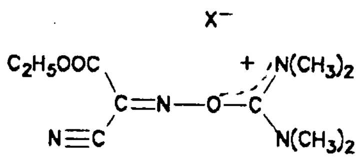

(I)

in welcher X für ein Anion steht.

Besonders bevorzugt sind Verbindungen der Formel I, worin X $BF_4^-$ oder $PF_6^-$ bedeutet. Insbesondere sei O-[(Cyano(ethoxycarbonyl)methyliden)amino]-1,1,3,3-tetramethyluronium-tetrafluorborat (TOTU) genannt.

Die erfindungsgemäßen Verbindungen zeichnen sich durch ein hohes Aktivierungspotential, einfache Herstellbarkeit (Eintopfreaktion) und gute Löslichkeit aus. Als Lösungsmittel werden bei der Herstellung ausschließlich Toluol und Aceton verwendet. Außerdem ist das in die Reaktion eingesetzte Natriumsalz des Oxims direkt aus Cyanessigsäureethylester zugänglich. Die bei der Aktivierung einer Carboxylgruppe mit Verbindungen der Formel I entstehenden Nebenprodukte Tetramethylharnstoff und 2-Hydroxyimino-2-cyanoessigsäureethylester sind gut in Wasser extrahierbar. Das Anion des Oxims ist darüberhinaus gelb gefärbt und kann daher bei der Extraktion des Reaktionsgemisches mit z.B. Bicarbonat gut detektiert werden. Ferner ist der Einsatz der Kupplungsreagenzien der Formel I sowohl bei der klassischen Peptidsynthese als auch bei der Festphasenpeptidsynthese möglich. Als Additiv zur Racemisierungsunterdrückung können verschiedene Reagenzien Verwendung finden wie z.B. 1-Hydroxybenzotriazol (HOBt), 3-Hydroxy-4-oxo-3,4-dihydro-1,2,3-benzotriazin (HOObt), 2-Hydroxypyridin-N-Oxid, 2-Hydroxy-2-cyanoessigsäureethylester und ähnliche.

Die Erfindung betrifft ferner ein Verfahren zur Herstellung von Verbindungen der Formel I, das dadurch gekennzeichnet ist, daß man Tetramethylharnstoff mit Oxalylchlorid oder Phosgen in Toluol umsetzt, das erhaltene Zwischenprodukt der Formel II nach Verdünnen der Lösung mit Aceton nacheinander mit dem Natriumsalz des gewünschten Anions und mit dem Natriumsalz von 2-Hydroxyimino-2-cyanoessigsäureethylester (M. Conrad, A. Schulze, Chem. Ber. 42 (1909) 735) versetzt, den Niederschlag absaugt, mit Aceton wäscht und das Produkt nach Zugabe von Isopropanol zu dem Filtrat kristallisiert.

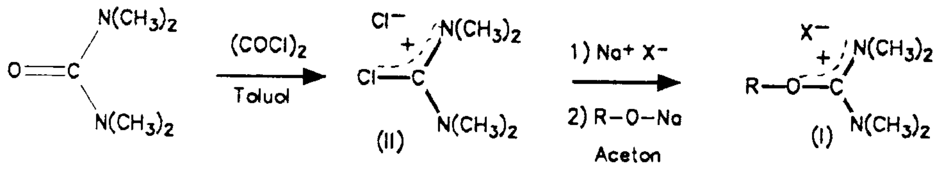


R ≡ 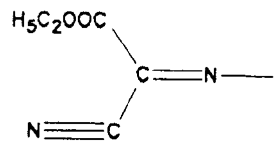

Kupplungsreaktionen mit Verbindungen der Formel I werden üblicherweise in geeigneten polaren aprotischen Lösungsmitteln wie z.B. Dimethylformamid (DMF), N-Methylpyrrolidon (NMP) oder Acetonitril, durchgeführt. Bei Verwendung eines Überschusses an Verbindung der Formel I kann auch in gemischt-wäßrigen Systemen gearbeitet werden.

Bei der Aktivierung einer Carbonsäure oder einer speziellen N-geschützten Aminosäure werden ein bis drei Äquivalente einer tertiären organischen Base zugesetzt.

In der Festphasenpeptidsynthese erfolgt bei Verwendung des erfindungsgemäßen Kupplungsreagenzes die Aktivierung der Aminosäure vorzugsweise separat. Bei Verwendung eines automatischen Peptidsynthesizers z.B. ABI 430 A (Fa. Applied Biosystems, U.S.A.) wird das Kupplungsreagenz entweder mit der Aminosäure als Festsubstanz oder als Lösung in definierter Menge in die Cartridges eingebracht. Die Aktivierung erfolgt durch Zugabe einer Base (1-2 Äquivalente). Gegegebenenfalls wird ein wie oben beschriebener racemisierungsunterdrückender Zusatz beigefügt. Die Voraktivierungszeit beträgt 3-30 Minuten.

Die Peptidkupplung erfolgt nach bekannten Methoden, wie z.B. in EP-A 271 865 (HOE 86/F 308) beschrieben.

Nachfolgend sind die Herstellung der Verbindungen der Formel I sowie einige Synthesebeispiele für die Anwendung gegeben.

**1. O-[Cyano(ethoxycarbonyl)methyliden)amino]-1,1,3,3-tetramethyl-uronium-tetrafluorborat (TOTU)**

121 ml (1 Mol) Tetramethylharnstoff werden in 300 ml Toluol gelöst. Dann werden unter gutem Rühren und unter Schutzgas (schwacher $N_2$-Strom) 86,4 ml (1 Mol) Oxalylchlorid gelöst in 100 ml Toluol in 20 Minuten zugetropft. Man rührt die Mischung bei 50°-55°C bis kein $CO_2$ mehr entweicht. Dann läßt man die erhaltene Suspension des gebildeten Zwischenprodukts auf Raumtemperatur abkühlen, gibt 600 ml trockenen Aceton hinzu und anschließend 104,3 g (0,95 Mol) Natriumtetrafluoroborat (feingepulvert). Man rührt die Suspension 1 h bei Raumtemperatur und addiert 156,9 g (0,95 Mol) Natriumsalz von Hydroxyiminocyanessigsäureethylester in Portionen. Die Mischung wird über Nacht gekühlt und dann der Niederschlag (Natriumchlorid und Produkt) abfiltriert. Das Filtrat wird am Rotationsverdampfer auf ca. 500 ml eingeengt und dann mit 1,2 l trockenem Isopropanol versetzt und 30 Minuten bei 40°C ausgerührt. Das ausgefallene Produkt wird abgesaugt und mit wenig Isopropanol (ca. 100 ml) nachgewaschen und im Exsikkator getrocknet. $N_1$ : 57,3 g Das Produkt/NaCl-Gemisch wird mit 400 ml trockenem Aceton 20 Minuten bei 40°C ausgerührt und warm filtriert. Man engt das Filtrat auf ca. 100 ml ein und versetzt mit 300 ml trockenem Isopropanol, rührt 20 Minuten bei 40°C und saugt das Produkt ab, wäscht mit Isopropanol und trocknet. $N_2$ : 43,6 g

Diese Prozedur wird noch zweimal mit dem NaCl/Produktgemisch durchgeführt $N_3$ : 31,5 g, $N_4$ : 5,3 g

Gesamtausbeute: $N_1$-$N_4$ : 137,6 g (44,1 %)

Schmp. 142-145°C, Zersetzung

| Elementaranalyse: | C | H | N | Cl | |
|---|---|---|---|---|---|
| berechnet: | 36,58 | 5,22 | 17,18 | 0 | % |
| gefunden: | 36,8 | 5,2 | 17,1 | <0,3 | % |

**A) Hydroxyiminocyanessigsäureethylester-Natriumsalz**

a) 1 Mol Hydroxyiminocyanessigsäureethylester werden in 400 ml Methanol (oder Ethanol) gelöst und unter Kühlung eine Lösung von 1 Mol NaOH in 300 ml Methanol zugetropft. Dann wird bis zur Trockene eingeengt und zweimal mit je 200 ml Toluol versetzt und zur Trockene eingeengt. Das erhaltene Natriumsalz wird nochmals im Exsikkator im Hochvakuum nachgetrocknet und dann fein gepulvert.
b) Direkt aus Cyanessigsäureethylester
Bei der Nitrosierung von Cyanessigsäureethylester mit Natriumnitrit und Essigsäure [M. Conrad, A. Schulze, Chem. Ber. 42, 735 (1909)] entsteht direkt das Natriumsalz. Dieses wird scharf getrocknet und mehrfach mit Essigester und Toluol in der Wärme suspendiert und zur Trockene eingeengt, um Essigsäure und Wasser zu trennen. Evtl. Kristallisation aus Essigester. Das Produkt sollte kein Natriumacetat enthalten (NMR-Kontrolle).

**2. Fmoc-D-Hyp-Gly-OtBu**

1,41 g Fmoc-D-Hyp-OH werden in 25 ml trockenem Acetonitril gelöst und dann 1,05 g H-Gly-OtBu sowie 0,445 g N-Hydroxypyridon zugegeben. Dann werden 1,31 g O-[(Cyano(ethoxycarbonyl)methyliden)amino]-1,1,3,3-tetramethyluronium-tetrafluoroborat (TOTU) in Protionen innerhalb von 10 Minuten eingetragen, sowie 1,03 ml Diisopropylethylamin in 15 ml DMF tropfenweise über 15 Minuten. Nach 1 h wird die Mischung eingeengt, der Rückstand in Essigester aufgenommen und mit Natriumchlorid-Lösung extrahiert. Die organische Phase wird über Natriumsulfat getrocknet und zur Trockene eingeengt. Das verbleibende Öl wird in wenig Ether aufgenommen und unter Rühren in Petrolether getropft. Der flockige Niederschlag wird abgesaugt und im Vakuum getrocknet.
Ausbeute: 671 mg
MS (FAB): 467 (M+H)

**3. Fmoc-Leu-Arg-Pro-Azagly-amid**

Zu einer Lösung von 136,5 mg H-Pro-Azagly-amid $HClO_4$ in 5 ml DMF werden 255 mg Fmoc-Leu-Arg-OH und bei 0°C unter Rühren 164 mg O-[(Cyano(ethoxycarbonyl)methyliden)amino]-1,1,3,3-tetramethyluronium-tetrafluoroborat (TOTU) sowie 0,065 ml N-Ethylmorpholin zugegeben. Man rührt noch 1 h bei 0°C und über Nacht bei Raumtemperatur. Dann wird von Unlöslichem abfiltriert und das Filtrat eingeengt. Man löst in 10 ml Pentanol und extrahiert dreimal mit 10 ml gesättigter wäßriger Bicarbonat-Lösung. Die organische Phase wird eingeengt, mit Diethylether verrieben und getrocknet.
Ausbeute: 260 mg
MS (FAB): 664 (M+H)

**4.**

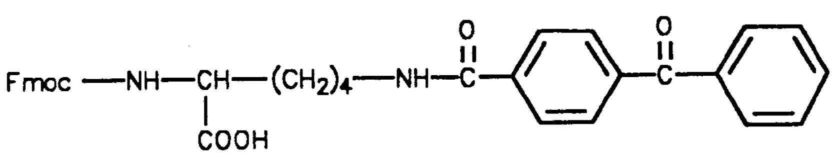

Zu einer Lösung von 4,52 g Benzophenon-4-carbonsäure in 200 ml DMF werden 2,22 g 2-Hydroxypyridin-N-oxid sowie 6,56 g TOTU gegeben und dann unter Rühren 5,23 ml Diisopropylethylamin zugetropft. Man läßt 30 Minuten bei Raumtemperatur reagieren und fügt dann 8,1 g Fmoc-Lys-OH x HCl zu. Anschließend läßt man noch 3 h bei Raumtemperatur reagieren und engt dann im Vakuum zur Trockene ein. Der Rückstand wird in 200 ml Ethylacetat aufgenommen und je dreimal mit je 20 ml $KHSO_4/K_2SO_4$-Lösung und $H_2O$ gewaschen. Die organische Phase wird über $Na_2SO_4$ getrocknet und zur Trockene eingeengt. Man nimmt in 50 ml Isopropanol auf und rührt in 500 ml Diisopropylether ein, saugt das ausgefallene Produkt ab und wäscht mit Diisopropylether. Nach Trocknung im Hochvakuum erhält man 4,76 g der Titelverbindung.
MS (FAB): 577(M+H)

**5. Synthese von Ac-D-Nal-pCl-D-Phe-D-Trp-Ser(tBu)-Tyr(tBu)-OtBu und Racemisierungstest**

Zu einer Lösung von 64,3 mg (0,25 mmol) Ac-D-Nal-OH und 244,3 mg H-pCl-D-Phe-D-Trp-Ser(tBu)-Tyr(tBu)-OtBu · Tos-OH in 5 ml DMF wurde bei 0 °C 0,25 mmol Kupplungsreagenz gegeben. Nach einstündigem Rühren bei 0 °C und über Nacht bei Raumtemperatur wurde die Mischung abfiltriert und das Filtrat im Vakuum eingeengt. Der Rückstand wurde mit konzentrierter Bicarbonatlösung behandelt. Der unlösliche Rückstand wurde abfiltriert, mit Wasser gewaschen, mit Kaliumsulfat/Kaliumhydrogensulfatlösung aufgenommen, filtriert, mit Wasser gewaschen und im Vakuum getrocknet.

Der Racemisierungsgrad des gekuppelten Ac-D-Nal-OH wurde mittels HPLC unter Verwendung einer Nukleosil 120-5 C8 250/8/4 Kolonne und Methanol/Wasser 7/3 als Lösungsmittel mit einer Flußrate von 1 ml/min gemessen. Elutionszeit: 37,7 min (Titelverbindung), 49,1 min racemisiertes Produkt.

Nachfolgende Tabelle zeigt die verwendeten Kupplungsverfahren und das Ergebnis des Racemisierungstests.

| Kupplungsverfahren | % L-Nal |
|---|---|
| DCC/HOObt/NEM | 1,21 |
| TOTU/HOObt/2 equ. NEM | 0,63 |

**6. Festphasensynthese von H-D-Arg-Arg-Pro-Hyp-Gly-Thi-Ser-D-Tic-Oic-Arg-OH**

Die Synthese erfolgte mit einem Peptidsynthesizer der Firma Applied Biosystems (Modell 430A) unter Verwendung von Fmoc Aminosäure und von uns modifizierten Programmen. Das Peptid wurde auf 1 g Fmoc-Arg(Mtr)-Wang-Harz (Beladung 0,4 mmol/g) entsprechend der nachfolgenden Schritte aufgebaut

1. Entfernung der Fmoc Schutzgruppe mit Piperidin/DMF
2. Waschen des Harzes mit DMF
3. Aufkuppeln der mit TOTU* aktivierten Aminosäure
4. Waschen des Harzes mit DMF

Diese Kupplungsschritte wurden für jede Aminosäure wiederholt bis das Peptid vollständig aufgebaut war.

1 mmol Fmoc-Aminosäure wurde in die Cartridges eingewogen und 2 ml 0,5 molare TOTU-Lösung in DMF, 2 ml DMF-Lösung (9,5 mol N-Hydroxypyridon und 1 mol Diisopropylethylamin) sowie 4 ml DMF hinzugefügt. Die Mischung wurde 1 min gerührt und dann in das Reaktionsgefäß gegeben. Die Kupplungszeit betrug 20-30 min. Folgende Aminosäurederivate wurden verwendet: Fmoc-Oic-OH, Fmoc-D-Tic-OH, Fmoc-Ser(tBu)-OH, Fmoc-Thi-OH, Fmoc-Gly-OH, Fmoc-Hyp-OH, Fmoc-Pro-OH, Fmoc-Arg(Mtr)-OH, Fmoc-D-Arg(Mtr)-OH. Nach beendeter Reaktion wurde die Fmoc-Gruppe durch Behandlung mit Piperidin in DMF entfernt. Danach wurde das Harz mit DMF, Dichlormethan, Isopropanol und Methyl-tert.Butyl-Ether gewaschen und im Vakuum getrocknet.

Ausbeute: 1,42 g Peptidharz.

Die Abspaltung des Peptids vom Harz erfolgte durch Behandlung mit 0,9 ml m-Kresol in 1,42 ml Dichlormethan und nachfolgender Zugabe von 14 ml Trifluoressigsäure und 1 ml Trimethylsilylbromid. Nach 2,5 Stunden wurde die Mischung in 200 ml kaltem Methyl-tert.Butyl-Ether filtriert. Der ausgefallene Rückstand wurde abfiltriert, in Wasser gelöst und die wäßrige Lösung mit Ethylacetat extrahiert um Verunreinigungen zu entfernen. Die wäßrige Lösung wurde anschließend mit Ionenaustauscherharz (R)Dowex IRA 93 (Acetatform) behandelt, filtriert und gefriergetrocknet. Das Rohpeptid (267 mg) wurde durch Chromatographie an (R)Sephadex LH 20 mit 10 %iger Essigsäure als Elutionsmittel weiter gereinigt.

**7. Synthese von [Boc-Phe-Val-NH-CH($CH_2$-$C_6H_5$)-CH(OH)]$_2$-**

828 mg [H-Val-NH-CH($CH_2$-$C_6H_5$)-CH(OH)]$_2$- · HCl wurden in 40 ml DMF gelöst. Dann wurden 959 mg Boc-Phe-OH hinzugefügt sowie 533 mg 2-Hydroxyimino-2-cyanoessigsäureethylester und bei 0 °C 1,23 g TOTU. Anschließend wurden 4,1 ml Diisopropylethylamino langsam hinzugegeben und 1 Stunde gerührt. Das Lösungsmittel wurde im Vakuum entfernt und der Rückstand zwischen Dichlormethan und Wasser verteilt. Die organische Phase wurde mit Kaliumhydrogensulfat/$K_2SO_4$-Lösung, gesättigte Bicarbonatlösung und Wasser gewaschen. Nach Trocknen über Natriumsulfat wurde die organische Phase zur Trockene eingedampft. Der Rückstand wurde in warmem Ethylacetat gelöst und mit Ether ausgefällt, abfiltriert mit Ether gewaschen und im Vakuum getrocknet.

Ausbeute: 896 mg

*) Die Voraktivierung der Aminosäuren wurde analog dem nachfolgenden Verfahren in den Cartridges durchgeführt

**Verwendete Abkürzungen:**

| | |
|---|---|
| Ac | Acetyl |
| Arg | Arginin |
| Azagly | Azaglycin |
| Boc | tert. Butoxycarbonyl |
| DCC | Dicyclohexylcarbodiimid |
| DMF | Dimethylformamid |
| Fmoc | 9-Fluorenylmethoxycarbonyl |
| Gly | Glycin |
| HOBt | 1-Hydroxybenzotriazol |
| HOObt | 3-Hydroxy-4-oxo-3,4-dihydro-1,2,3-benzotriazin |
| D-Hyp | D-Hydroxyprolin |
| Leu | Leucin |
| Mtr | 2,3,5-Trimethyl-4-methoxyphenylsulfonyl |
| D-Nal | D-2-Naphthylalanin |
| NEM | N-Ethylmorpholin |
| Oic | cis-endo Octahydroindol |
| OtBu | O-tert.Butyl |
| D-Phe | Phenylalanin |
| Pro | Prolin |
| Ser | Serin |
| Thi | 2-Thienylalanin |
| D-Tic | 1,2,3,4-Tetrahydroisochinolin-3-yl-carbonyl |
| Tos | Toluol-4-sulfonyl |
| D-Trp | D-Tryptophan |
| Tyr | Tyrosin |
| Val | Valin |

## Patentansprüche

**Patentansprüche für folgende Vertragsstaaten : AT, BE, CH, DE, DK, FR, GB, GR, IT, LI, LU, NL, SE**

1. Verbindung der Formel I

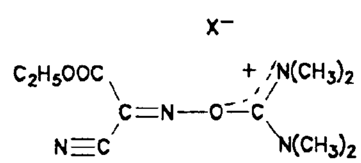

(I)

in welcher X für ein Anion steht.

2. Verbindung der Formel I gemäß Anspruch 1, dadurch gekennzeichnet, daß X $BF_4^-$ oder $PF_6^-$ bedeutet.

3. Verbindung der Formel I gemäß Anspruch 1 oder 2, dadurch gekennzeichnet, daß X $BF_4^-$ bedeutet.

4. Verfahren zur Herstellung einer Verbindung der Formel I gemäß den Ansprüchen 1 bis 3, dadurch gekennzeichnet, daß man Tetramethylharnstoff mit Oxalylchlorid oder Phosgen in Toluol umsetzt, das erhaltene Produkt der Formel II

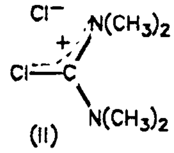

(II)

nach Verdünnen der Lösung mit Aceton nacheinander mit dem Natriumsalz des gewünschten Anions und mit dem Natriumsalz von 2-Hydroxyimino-2-cyanoessigsäureethylester versetzt, den Niederschlag absaugt, mit Aceton wäscht und das Produkt nach Zugabe von Isopropanol zu dem Filtrat kristallisiert.

5. Verwendung einer Verbindung der Formel I gemäß den Ansprüchen 1 bis 3 als Kupplungsreagenz für die Peptidsynthese.

**Patentansprüche für folgenden Vertragsstaat : ES**

1. Verfahren zur Herstellung einer Verbindung der Formel I

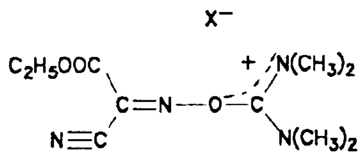

(I)

in welcher X für ein Anion steht, dadurch gekennzeichnet, daß man Tetramethylharnstoff mit Oxalylchlorid oder Phosgen in Toluol umsetzt, das erhaltene Produkt der Formel II

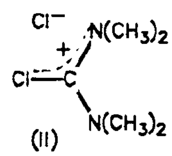

(II)

nach Verdünnen der Lösung mit Aceton nacheinander mit dem Natriumsalz des gewünschten Anions und mit dem Natriumsalz von 2-Hydroxyimino-2-cyanoessigsäureethylester versetzt, den Niederschlag absaugt, mit Aceton wäscht und das Produkt nach Zugabe von Isopropanol zu dem Filtrat kristallisiert.

2. Verfahren gemäß Anspruch 1, dadurch gekennzeichnet, daß X $BF_4^-$ oder $PF_6^-$ bedeutet.

3. Verfahren gemäß Anspruch 1 oder 2, dadurch gekennzeichnet, daß X $BF_4^-$ bedeutet.

**Claims**

**Claims for the following Contracting States : AT, BE, CH, DE, DK, FR, GB, GR, IT, LI, LU, NL, SE**

1. A compound of the formula I

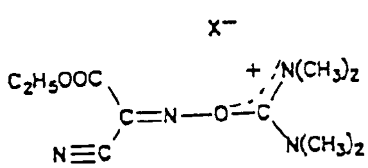

(I)

in which X is an anion.

2. A compound of the formula I as claimed in claim 1, wherein X is $BF_4^-$ or $PF_6^-$.

3. A compound of the formula I as claimed in claim 1 or 2, wherein X is $BF_4^-$.

4. A process for preparing a compound of the formula I as claimed in claims 1 to 3, which comprises reacting tetramethylurea with oxalyl choride or phosgene in toluene, mixing the resulting product of the formula II,

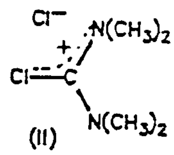

(II)

after dilution of the solution with acetone, successively with the sodium salt of the desired anion and with the sodium salt of ethyl 2-hydroxyimino-2-cyanoacetate, filtering off the precipitate with suction and washing it with acetone, and crystallizing the product after addition of isopropanol to the filtrate.

5. The use of a compound of the formula I as claimed in claims 1 to 3 as coupling reagent for peptide synthesis.

**Claims for the following Contracting State : ES**

1. A process for preparing a compound of the formula I

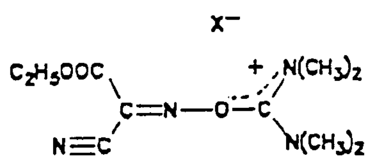

(I)

in which X is an anion, which comprises reacting tetramethylurea with oxalyl chloride or phosgene in toluene, mixing the resulting product of the formula II,

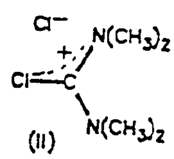

(II)

after dilution of the solution with acetone, successively with the sodium salt of the desired anion and with the sodium salt of ethyl 2-hydroxyimino-2-cyanoacetate, filtering off the precipitate with suction and washing it with acetone, and crystallizing the product after addition of isopropanol to the filtrate..

2. A process as claimed in claim 1, wherein X is $BF_4^-$ or $PF_6^-$.

3. A process as claimed in claim 1 or 2, wherein X is $BF_4^-$.

## Revendications

### Revendications pour les Etats contractants suivants : AT, BE, CH, DE, DK, FR, GB, GR, IT, LI, LU, NL, SE

1. Composé de formule I

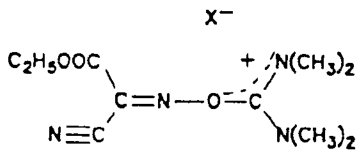

(I)

dans laquelle X représente un anion.

2. Composé de formule I selon la revendication 1, caractérisé en ce que X représente $BF_4^-$ ou $PF_6^-$.

3. Composé de formule I selon la revendication 1 ou 2, caractérisé en ce que X représente $BF_4^-$.

4. Procédé pour la préparation d'un composé de formule I selon les revendications 1 à 3, caractérisé en ce que l'on fait réagir de la tétraméthylurée avec du chlorure d'oxalyle ou du phosgène dans due toluène, on ajoute au produit de formule II

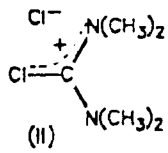

(II)

obtenu, après dilution de la solution avec de l'acétone, successivement le sel sodique de l'anion désiré et le sel sodique du 2-hydroxyimino-2-cyanoacétate d'éthyle, on sépare le précipité par filtration sous aspiration, on le lave avec de l'acétone, et on fait cristalliser le produit après addition d'isopropanol au filtrat.

5. Utilisation d'un composé de formule I selon les revendications 1 à 3, en tant que réactif de couplage pour la synthèse de peptides.

### Revendications pour l'Etat contractant suivant : ES

1. Procédé pour la préparation d'un composé de formule I

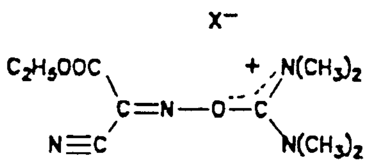

(I)

dans laquelle X représente un anion, caractérisé en ce que l'on fait réagir de la tétraméthylurée avec du chlorure d'oxalyle ou du phosgène dans du toluène, on ajoute au produit de formule II

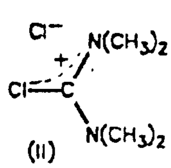

(II)

obtenu, après dilution de la solution avec de l'acétone, successivement le sel sodique de l'anion désiré et le sel sodique du 2-hydroxyimino-2-cyanoacétate d'éthyle, on sépare le précipité par filtration sous aspiration, on le lave avec de l'acétone, et on fait cristalliser le produit après addition d'isopropanol au filtrat.

**2.** Procédé selon la revendication 1, caractérisé en ce que X représente $BF_4^-$ ou $PF_6^-$.

**3.** Procédé selon la revendication 1 ou 2, caractérisé en ce que X représente $BF_4^-$.